# EUROPÄISCHE PATENTSCHRIFT

(11) **EP 2 197 379 B1**
(45) Veröffentlichungstag und Bekanntmachung des Hinweises auf die Patenterteilung: **20.02.2013**
(21) Anmeldenummer: 08785271.1
(22) Anmeldetag: 31.07.2008
(51) Int. Cl.: A61B 18/14, A61B 17/32, A61B 17/00, A61M 25/00, A61B 1/018

(54) **ELEKTROCHIRURGISCHES INSTRUMENT UND EIN ENDOSKOP MIT ENTSPRECHENDEM INSTRUMENT**
ELECTROSURGICAL INSTRUMENT, AND AN ENDOSCOPE WITH A CORRESPONDING INSTRUMENT
INSTRUMENT ÉLECTROCHIRURGICAL ET ENDOSCOPE ÉQUIPÉ D'UN TEL INSTRUMENT

(30) Priorität: 29.08.2007 DE 102007040842
(43) Veröffentlichungstag der Anmeldung: 23.06.2010
(73) Patentinhaber: ERBE Elektromedizin GmbH, 72072 Tübingen (DE)
(72) Erfinder: FISCHER, Klaus, 72202 Nagold (DE); VOIGTLÄNDER, Matthias, 72810 Gomaringen (DE); SCHÄLLER, Daniel, 72070 Tübingen (DE); SZYRACH, Mara, 72070 Tübingen (DE); BLOBEL, Lars, 72119 Ammerbuch-Entringen (DE); SIGLE, Irina, 72116 Mössingen (DE)
(74) Vertreter: Bohnenberger, Johannes
(86) Internationale Anmeldenummer: PCT/EP2008/006330
(87) Internationale Veröffentlichungsnummer: WO 2009/030324

(56) Entgegenhaltungen:
- WO-A-2007/058816
- DE-A1-102006 006 052
- DE-A1-102007 009 725

## Beschreibung

Die Erfindung betrifft ein elektrochirurgisches Instrument nach Anspruch 1 und ein Endoskop mit entsprechendem Instrument gemäß dem Anspruch 13.

Aus der EP 1 293 169 B1 ist ein Endoskop bekannt. Es umfasst mindestens einen Arbeitskanal, in den sich ein Schutzschlauch einbringen lässt. Dieser Schutzschlauch dient zur Führung von Instrumenten. Das Endoskop könnte bei einer endoskopischen submukosalen Dissektion (ESD) ein nadelförmiges Schneidinstrument aufnehmen. Die EP 1 293 169 B1 zeigt ein elektrochirurgisches Instrument zur Koagulation. Instrumente zur Dissektion oder Resektion, wobei mittels Hochfrequenzchirurgie Gewebe präzise geschnitten und bei Bedarf koaguliert wird, sind ebenfalls bekannt.

Bei einer typischen ESD wird Flüssigkeit in die Submukosa injiziert, um die Mukosa von der Muskularis abzuheben und einen ausreichend großen Hohlraum für die Resektion zu erhalten. Bei einer ESD werden bevorzugt nadelförmige Schneidinstrumente eingesetzt.

Bei endoskopischen Dissektionen, insbesondere mittels nadelförmigen Instrumenten, stellt die Bestimmung und Einstellung der Position der Instrumentenspitze relativ zu dem Schutzschlauch, aber auch relativ zu dem Ende des Arbeitskanals, ein großes Problem dar. Bei einer fehlerhaften Bestimmung der Position oder bei einer schlechten Einstellung dieser, kann es zu Verletzungen oder ungewollten Perforationen, insbesondere der Muskularis, kommen.

Für eine einfache Handhabung des Instruments ist es mitunter hilfreich, wenn verschiedene, fest vorgegebene Positionen des Schneidinstruments relativ zum Schutzschlauch bereitgestellt werden. Bei der ESD benötigt man beispielsweise eine möglichst kurze Nadellänge für das Setzen von Markierungspunkten und eine möglichst lange Nadellänge für die Zircumcision.

In der Praxis hängt die präzise Bestimmung und Einstellung der Position des Schneidinstruments, beispielsweise einer aktiven Schneidelektrode, stark von der visuellen Kontrolle des die Operation durchführenden Arztes ab.

Eine präzise Voreinstellung oder das Bereitstellen von bestimmten, vorab definierten Positionen scheitert an zahlreichen Problemen. Zum einen muss nicht nur die Relativposition der Instrumentenspitze zum Schutzschlauch, sondern auch die Relativposition der Spitze des Schutzschlauches zum Arbeitskanal berücksichtigt werden. Je nach verwendetem Material kann der Schutzschlauch bei der Ausübung von Kraft gedehnt werden, wodurch sich die Länge des Schlauches und somit die Position der Instrumentenspitze ändert. Des Weiteren können die ständige Bewegung des flexiblen Arbeitskanals sowie die Bewegung des Schutzschlauches innerhalb des Arbeitskanals die Position eines vorab innerhalb des Schutzschlauches positionierten Instruments verändern.

Hinzu kommt, dass selbst bei einer sicheren Bestimmung der Relativposition des Instruments zum Schutzschlauch der Schutzschlauch durch leichten Druck in das Gewebe eindringt und somit die maximal erzielbare Schneidtiefe verändert. Daher sollte auch die Position des distalen Endes des Schutzschlauches genau bestimmbar sein.

Bei einer visuellen Kontrolle kommt erschwerend hinzu, dass das distale Ende des chirurgischen Instruments aufgrund der Verschmutzung durch Geweberückstände oft nicht sichtbar ist und eine weitere Anwendung des Gerätes zumindest erschwert wird. Möchte man diese Geweberückstände entfernen, so bleibt einem mitunter nichts anderes übrig, als das Instrument mit oder ohne Schutzschlauch aus dem Arbeitskanal heraus zu nehmen. Häufig wird dann das Instrument durch ein neues, steriles Instrument ersetzt. Die Kosten hierfür sind nicht unerheblich.

Der Erfindung liegt daher die Aufgabe zu Grunde, ein elektrochirurgisches Instrument bereit zu stellen, das einfach und sicher handhabbar ist. Des Weiteren soll ein entsprechendes Endoskop angegeben werden.

Diese Aufgabe wird erfindungsgemäß durch ein elektrochirurgisches Instrument nach Anspruch 1 bzw. durch ein Endoskop nach Anspruch 13 gelöst.

Insbesondere wird die Aufgabe durch ein elektrochirurgisches Instrument, umfassend einen in einen Arbeitskanal eines Endoskops einführbaren Schutzschlauch und eine Elektrode, die innerhalb des Schutzschlauches mittels eines Bedienelements in eine distale Richtung und eine proximale Richtung bewegbar ist, gelöst, wobei die Elektrode durch eine Bewegung in die distale Richtung in eine ausgefahrene Position bringbar ist, in der die Elektrode zumindest teilweise über ein distales Ende des Schutzschlauches heraus ragt. Das distale Ende des Schutzschlauches umfasst ein Abschlusselement mit einer Öffnung für den Durchlass der Elektrode, wobei die Öffnung des Abschlusselements zur Führung und/oder Reinigung der Elektrode einen maximalen Innendurchmesser d1 aufweist, der geringer ist als der Innendurchmesser d2 des Schutzschlauches.

Das besagte elektrochirurgische Instrument ist dadurch gekennzeichnet, dass ein Fixierelement am distalen Ende des Schutzschlauches derart ausgebildet ist, dass das Fixierelement eine definierte Haltekraft induziert, die einer Bewegung der Elektrode in der distalen und/oder proximalen Richtung entgegen wirkt. Das Fixierelement kann entweder direkt an der Elektrode ansetzen oder über das Bedienelement auf diese einwirken. Mittels des Fixierelements wird die Elektrode gegenüber dem Schutzschlauch derart festgesetzt, dass ein ungewolltes Verschieben der Elektrode relativ zum Schutzschlauch verhindert wird. Somit kann eine vorab eingestellte Position der Elektrode relativ zum distalen Ende des Schutzschlauches problemlos beibehalten werden.

Das Fixierelement umfasst mindestens ein elastisches Element, das innerhalb des Schutzschlauches angeordnet ist. Dieses elastische Element kann die Haftkraft zwischen Elektrode oder Bedienelement und Schutzschlauch induzieren.

Vorzugsweise ist die induzierte Haftkraft derart bemessen, dass sie geringfügig höher ist als die Reibungs- oder Adhäsionskräfte von Schutzschlauch und Endoskop. Somit kann der Schutzschlauch innerhalb des Arbeitskanals des Endoskops problemlos verschoben werden, ohne dass es zu einer Änderung der Relativposition der Elektrode zum distalen Ende des Schutzschlauches kommt.

Die Elektrode bzw. das Schneidinstrument lässt sich also innerhalb des Schutzschlauches mittels eines Bedienelements vor- und zurückbewegen. Für das Erreichen einer ausgefahrenen Position der Elektrode wird diese so lange in die distale Richtung (bezüglich des Schutzschlauches) bewegt, bis zumindest ein Abschnitt der Elektrode über das distale Ende des Schutzschlauches heraus ragt.

Ein wesentlicher Punkt der Erfindung besteht darin, dass das distale Ende des Schutzschlauches eine Verengung mit einer Öffnung aufweist, durch die die Elektrode zum Erreichen der ausgefahrenen Position geführt wird. Die Öffnung dient hier insbesondere dazu, die Elektrode zu führen und/oder zu reinigen. Die besagte Öffnung hat einen maximalen Innendurchmesser, der geringer ist als der Innendurchmesser des Schutzschlauches. Wenn die Öffnung kreisförmig ausgebildet ist, entspricht der maximale Innendurchmesser der Öffnung gleich dem Durchmesser. Alternativ kann die Öffnung jede beliebige zweckdienliche Form aufweisen. Beispielsweise kann sie elliptisch, quadratisch, rechteckig usw. sein. Für die Erfindung ist es lediglich notwendig, dass die Öffnung des Abschlusselements im Querschnitt den Innenraum des Schlauches beschränkt.

In einer Ausführungsform umfasst die Elektrode ein Stopperelement, das mit dem Abschlusselement derart zusammenwirkt, dass es die Bewegung der Elektrode in distaler Richtung beschränkt. Das Stopperelement stellt also zusammen mit dem Abschlusselement eine mechanische Beschränkung der Bewegung der Elektrode bereit. Hierdurch ist die Ausfahrlänge der Elektrode in distaler Richtung beschränkt.

In einer Ausführungsform ist das Stopperelement zylindrisch ausgebildet. Das Stopperelement ist also beispielsweise dem Innenraum des Schutzschlauches entsprechend ausgebildet und in diesem hin- und herbewegbar. Diese Ausbildung des Stopperelements ist besonders vorteilhaft, wenn der Querschnitt der Elektrode wesentlich geringer ist als der des Schutzschlauches. Das Stopperelement ermöglicht dann eine kräftefreie und/oder gezielte Führung der Elektrode in die Öffnung des Abschlusselements. Die mechanische Beschränkung der Bewegung der Elektrode kann in einfacher Weise durch ein zumindest abschnittweises Anstoßen des Stopperelements an Teile des Abschlusselements gewährleistet werden.

In einer Ausführungsform ist ein Federelement, insbesondere eine Schraubenfeder, zwischen dem Stopperelement und dem Abschlusselement angeordnet, wobei das Federelement in der ausgefahrenen Position der Elektrode eine Kraft in die proximale Richtung injiziert. Durch die Bewegung der Elektrode beispielsweise durch das Bedienelement in distale Richtung wird also eine Federkraft aufgebaut, die dieser Bewegung entgegen wirkt. Das Federelement wird dazu genutzt, einen automatischen Einzugsmechanismus der Elektrode in einen Bereich innerhalb des Schutzschlauches bereit zu stellen. Somit kann ein ungewolltes Schneiden des Gewebes verhindert werden. Die Federkraft beschränkt ebenfalls die Bewegbarkeit der Elektrode in distaler Richtung.

In einer Ausführungsform ist die Öffnung des Abschlusselements der Elektrode dem Querschnitt der Elektrode formentsprechend ausgebildet. Die Öffnung ist also im Querschnitt an den Querschnitt der Elektrode angepasst, so dass insbesondere bei einem Zurückführen der Elektrode aus der ausgefahrenen Position in das Innere des Schutzschlauches ein Abstreifen von an der Elektrode angehafteten Ablagerungen, insbesondere Gewebeablagerungen, erfolgt. Der operative Eingriff kann so wesentlich effizienter gestaltet werden, da ein Auswechseln der Elektrode mit zugehörigen Bedienelementen unnötig ist. Aufgrund dieses einfachen Reinigungsprozesses kann auch die visuelle Kontrolle bei der Führung der Elektrode verbessert werden.

In einer Ausführungsform ist die Öffnung des Abschlusselements zur kraftfreien Durchführung der Elektrode ausgebildet.

In einer Ausführungsform kann die Elektrode eine Nadel umfassen. Für eine ESD ist eine derartige Ausgestaltung des Instruments vorteilhaft.

Insbesondere kann für diesen Anwendungsfall innerhalb der Elektrode eine Kapillarleitung zum Injizieren von Flüssigkeit vorgesehen sein. Beispielsweise kann bei einer ESD Flüssigkeit über die besagte Kapillarleitung in die Submukosa injiziert werden, um diese von der Muskularis abzuheben. Ein zeitaufwändiges Wechseln des Instruments bei der Operation kann unterbleiben.

In einer Ausführungsform umfasst der Schutzschlauch mindestens eine insbesondere ringförmig ausgebildete Markierung an der Außenseite des Schutzschlauches nahe dessen distalem Ende. Diese Markierungen können mit einem vordefinierten Abstand zur distalen Spitze des Schutzschlauches angeordnet sein und das optische Erfassen der Position des Schutzschlauches, insbesondere dessen Endes, erleichtern. Insbesondere kann festgestellt werden, wie weit das Ende des Schutzschlauches an bzw. in ein zu behandelndes Gewebe heran bzw. hinein ragt.

In einer weiteren Ausführungsform umfasst das elektrochirurgische Element eine Bedieneinheit zur Einstellung der Position der Elektrode relativ zum distalen Ende des Schutzschlauches. Die Bedieneinheit erleichtert also das Einstellen der Relativposition.

Vorzugsweise umfasst die Bedieneinheit mindestens eine Sperreinrichtung, mit der das Bedienelement und somit die Elektrode in mindestens einer Einstellposition festgesetzt werden kann. Eine einmal eingestellte Position der Elektrode relativ zum distalen Ende des Schutzschlauches kann so einfach beibehalten werden.

In einer weiteren Ausführungsform weist die Bedieneinheit mindestens zwei Einstellpositionen auf, wobei in einer ersten Einstellposition die Elektrode mindestens teilweise über ein distales Ende des Schutzschlauches herausragt und in einer zweiten Einstellposition die Elektrode innerhalb des Schutzschlauches liegt. Derartige, vorab festgelegte Einstellpositionen, können die Handhabung des elektrochirurgischen Instruments wesentlich erleichtern. Selbst bei geringem Sichtkontakt kann ein sicheres Einstellen der Relativpositionen gewährleistet werden.

Des Weiteren wird die oben aufgeführte Aufgabenstellung durch ein Endoskop mit mindestens einem Arbeitskanal und einem elektrochirurgischen Instrument, wie dies vorab beschrieben wurde, gelöst. Die Vorteile eines derartigen Endoskops ergeben sich in ähnlicher Weise, wie dies bereits anhand des elektrochirurgischen Instruments erläutert wurde.

Nachfolgend wird die Erfindung mittels einiger Ausführungsbeispiele beschrieben, die mittels Abbildungen näher erläutert werden. Hierbei zeigen
- - Fig. 1: einen schematischen Schnitt durch ein Endoskop;
- - Fig. 2: ein nadelförmiges Instrument mit Schutzschlauch bei einer ESD;
- - Fig. 3 u. 4: einen Schnitt durch das distale Ende eines ersten elektrochirurgischen Instruments;
- - Fig. 5: einen Schnitt durch das distale Ende eines zweiten elektrochirurgischen Instruments mit ausgefahrener Elektrode;
- - Fig. 6: einen Schnitt durch das distale Ende des zweiten elektrochirurgischen Instruments mit eingefahrener Elektrode;
- - Fig. 7 u. 8: einen Schnitt durch das distale Ende eines dritten, erfindungsgemäßen elektrochirurgischen Instruments mit Elastomerring;
- - Fig. 9: einen Schnitt durch eine erfindungsgemäße Nadelelektrode;
- - Fig. 10 u. 11: die schematische Darstellung eines Teilabschnitts eines erfindungsgemäßen elektrochirurgischen Instruments bei einer ESD;
- - Fig. 12: eine schematisierte endoskopische Sicht auf das erfindungsgemäße elektrochirurgische Instrument; und
- - Fig. 13 u. 14: eine Bedieneinheit für ein erfindungsgemäßes elektrochirurgisches Instrument.

In der nachfolgenden Beschreibung werden für gleiche und gleich wirkende Teile dieselben Bezugsziffern verwendet.

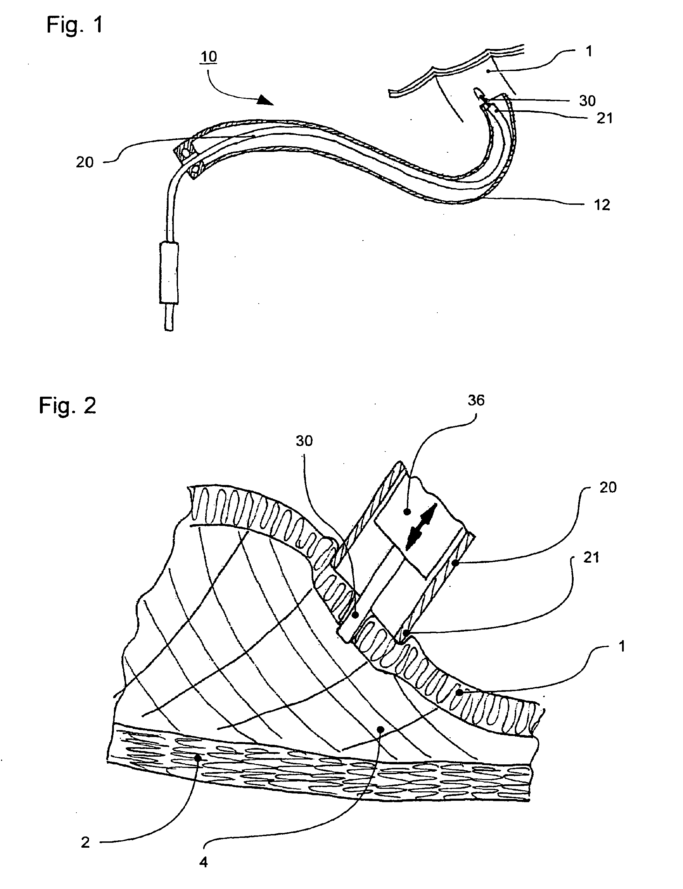

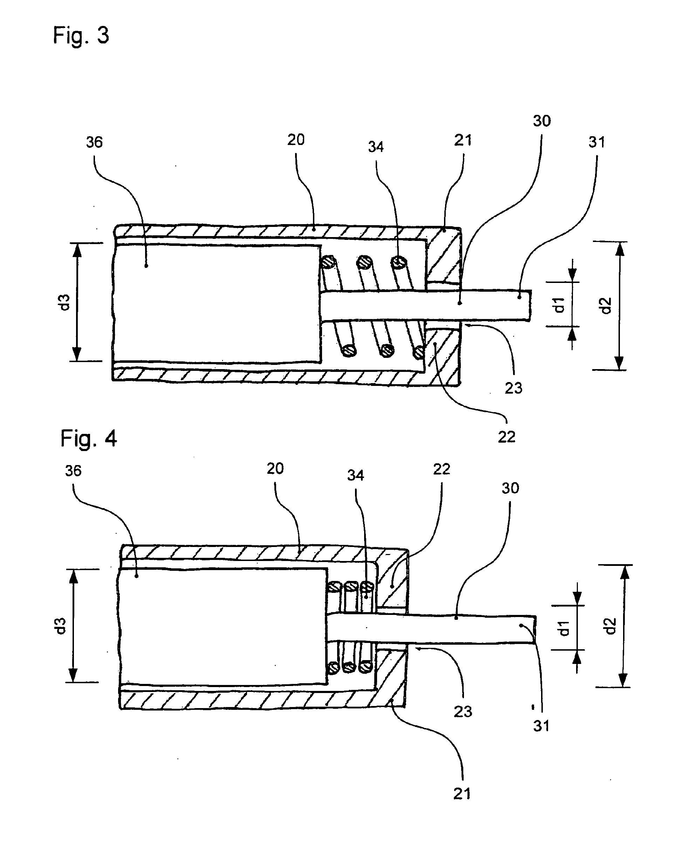

Die Fig. 1 stellt stark schematisierend der Schnitt durch ein Endoskop 10 dar. Das Endoskop 10 umfasst einen Arbeitskanal 12, in den ein Schutzschlauch 20 eingeführt ist. Im Inneren des Schutzschlauches 20 befindet sich eine Führungseinrichtung 36 (nicht gezeigt), die mit einer Nadelelektrode 30 in Wirkverbindung steht. Mittels der Führungseinrichtung 36 lässt sich die Nadelelektrode 30 innerhalb des Schutzschlauches 20 in distaler und proximaler Richtung verschieben. In dem Endoskop 10 der Fig. 1 befindet sich die Nadelelektrode 30 in einer ausgefahrenen Position, bei der die Spitze 31 (vgl. Fig. 3 und 4) der Nadelelektrode 30 über das Distalende 21 des Schutzschlauches 20 heraus ragt.

In der Fig. 1 ist die Außenwand eines Hohlorgans, insbesondere dessen Mukosa 1 schematisch dargestellt, in die die Nadelelektrode 30 eingeführt werden soll.

Die Fig. 1 verdeutlicht, dass die Positionsbestimmung der Nadelelektrode 30 aufwändig ist, da zum einen der Arbeitskanal 12, zum anderen der Schutzschlauch 20 biegbar ist. Eine starke Krümmung dieser verändert nicht nur die Reibungseigenschaften der Vorrichtung, sondern kann auch zu einer Veränderung der Position der Nadelelektrode 30 absolut oder relativ zum Arbeitskanal 12 und/oder zum Schutzschlauch 20 führen.

Fig. 2 zeigt ein Hohlorgan im Querschnitt. Im Zuge einer ESD wurde Flüssigkeit 4 über die Nadelelektrode 30 in die Mukosa 1 eingebracht und diese von der Muskularis 2 des Hohlorgans gelöst. Die Flüssigkeit 4 bildet einen Zwischenraum aus, der einen ausreichend großen Abstand bei der Resektion zwischen den beiden Schichten gewährleistet.

Die Fig. 2 verdeutlicht, dass die visuelle Positionsbestimmung bei herkömmlichen Endoskopen 10 schwierig und mitunter unmöglich ist. Da in dem in der Fig. 2 dargestellten Beispiel der Schutzschlauch 20 des Endoskops 10 teilweise in das weiche Gewebe der Mukosa 1 eingedrungen ist, ist das Distalende 21 des Schutzschlauches 20 durch das Gewebe und sich ausbildende Falten verdeckt. Eine visuelle Erfassung des Distalendes 21 ist daher nicht möglich. Entsprechend problematisch gestaltet sich die Bestimmung der Position der Nadelelektrode 30, die durch eine Führungseinrichtung 36 in distaler und proximaler Richtung verschoben werden kann. In einer derartigen Konstellation kann die Absolutposition auch bei bekannter Relativposition der Nadelelektrode 30 zum Schutzschlauch 20 optisch nicht bestimmt werden.

Die Fig. 3 zeigt das Distalende 21 eines Schutzschlauches 20. Der Schutzschlauch 20 umfasst ein Abschlusselement 22, das sich vom distalen Ende 21 des Schutzschlauches 20 im Wesentlichen senkrecht zur Außenhaut des Schutzschlauches 20 in dessen Innenraum erstreckt. Das Abschlusselement 22 bildet eine Öffnung 23 aus, die einen maximalen Innendurchmesser d1 hat, der wesentlich geringer ist als der Innendurchmesser d2 des Schutzschlauches 20. Somit ist das Distalende 21 des Schutzschlauches verengt. Die Öffnung 23 erstreckt sich parallel zu der Längsrichtung einer länglichen Nadelelektrode 30 und ist zur reibungsfreien Durchführung dieser geeignet.

Die Nadelelektrode 30 ist auf eine Führungseinrichtung 36 mittig aufgesetzt, die sich ebenfalls innerhalb des Schutzschlauches 20 befindet und ein Bewegen der Nadelelektrode 30 in distaler und proximaler Richtung ermöglicht, wodurch sich die Relativposition der Spitze 31 der Nadelelektrode 30 zum Schutzschlauch 20, insbesondere zu dessen Distalende 21 verändert.

Die Führungscinrichtung 36 ist im Wesentlichen zylinderförmig ausgebildet und weist einen Innendurchmesser d3 auf, der größer als der Innendurchmesser d1 der Öffnung 23, und kleiner als der Innendurchmesser d2 des Schutzschlauches 20 ist. Somit lässt sich die Führungseinrichtung 36 mit geringem Kraftaufwand innerhalb des Schutzschlauches 20 bewegen. Der Innendurchmesser d3 der Führungseinrichtung 36 und die Anbringung der Nadelelektrode 30 an diesem ist derart gestaltet, dass ein problemloses Einführen der Nadelelektrode 30 in die Öffnung 23 möglich ist. Aufgrund der Differenz zwischen dem Innendurchmesser d1 der Öffnung 23 und dem Innendurchmesser d3 der Führungseinrichtung 36 bildet das Abschlusselement 22 zusammen mit der zylindrischen Führungseinrichtung 36 ein Stopperelement, das die Bewegung der Führungseinrichtung 36 in distaler Richtung mechanisch beschränkt.

Zwischen dem Abschlusselement 22 und der Führungseinrichtung 36 ist ein Federelement 34 angeordnet. Bei dem vorliegenden Ausführungsbeispiel handelt es sich um eine Spiralfeder, die die Nadelelektrode 30 abschnittsweise umgibt. Je weiter man die Nadelelektrode 30 und die Führungseinrichtung 36 in distaler Richtung bewegt, um so stärker spannt sich das Federelement 34, wodurch eine Kraft aufgebaut wird, die der Bewegungsrichtung entgegen wirkt. Sobald der operierende Arzt keine Kraft auf die Führungseinrichtung 36 ausübt, so wird die Nadelelektrode 30, bedingt durch die durch das Federelement 34 induzierte Kraft eingefahren. Man kann beispielsweise drei Positionen der Nadelelektrode 30 bzw. der entsprechend vorliegenden Nadellänge (Distanz zwischen Distalende 21 des Schutzschlauches 20 und der Spitze 31 der Nadelelektrode 30) unterscheiden:
- Nadellänge ₌ 0, wenn keine Kraft über die Führungseinrichtung 36 aufgewandt wird;
- kurze Nadellänge bei geringem Kraftaufwand (vgl. Fig. 3);
- maximale Nadellänge bei hohem Kraftaufwand (vgl. Fig. 4).

Die durch das Federelement 34 induzierte Kraft hilft dem Arzt bei der Bedienung der Nadelelektrode 30 und bei der Bestimmung deren Länge. Ist der Arzt an das Instrument gewöhnt, so kann er anhand der für die Bedienung der Nadelelektrode 30 nötigen Kraft abschätzen, wie weit die Spitze 31 der Nadelelektrode 30 über das Distalende 21 des Schutzschlauches 20 heraus ragt.

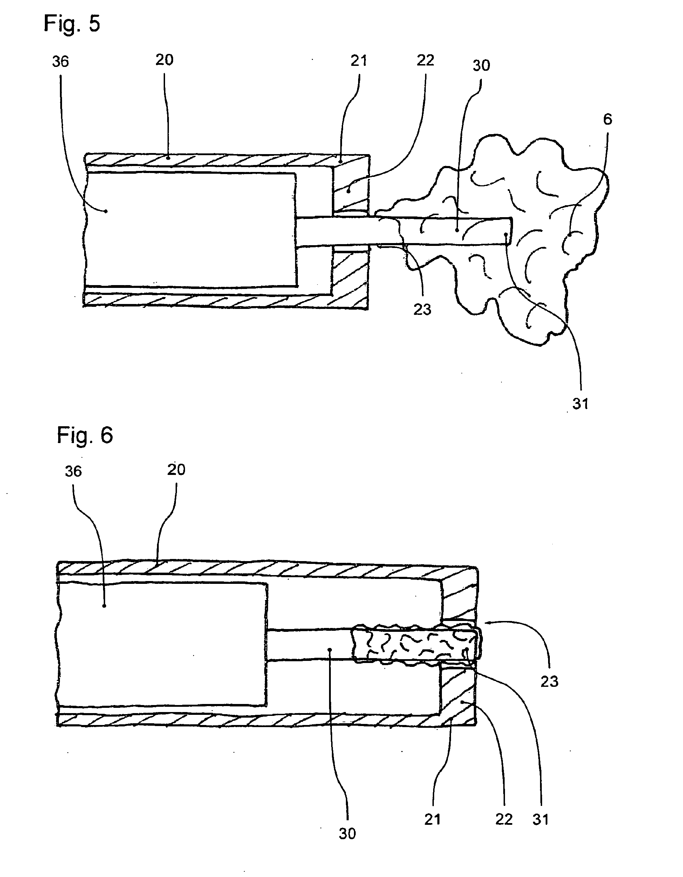

Die Fig. 5 und 6 verdeutlichen einen weiteren Vorteil des Abschlusselements 22. Auch hier sind das Distalende 21 des Schutzschlauches 20, die Nadelelektrode 30 sowie die Führungseinrichtung 36 dargestellt. Auf eine Darstellung des Federelements 34 wurde verzichtet.

Aufgrund der geringen Differenz zwischen dem Innendurchmesser d1 der Öffnung 23 und dem Innendurchmesser der zylindrisch ausgebildeten Nadelelektrode 30 kann das Abschlusselement zur Reinigung der Nadelelektrode 30 eingesetzt werden. Gewebe 6, das sich bei der Operation an den Seitenwänden der Nadelelektrode 30 festsetzt, kann beim Einfahren der Nadelelektrode 30 in das Innere des Schutzschlauches 20 am Abschlusselement 22 abgestreift werden. Bei erneutem Ausfahren der Nadelelektrode 30 ist deren Spitze 31 gut zu erkennen, wodurch eine effektive visuelle Kontrolle der Position der Nadelelektrode 30 möglich wird.

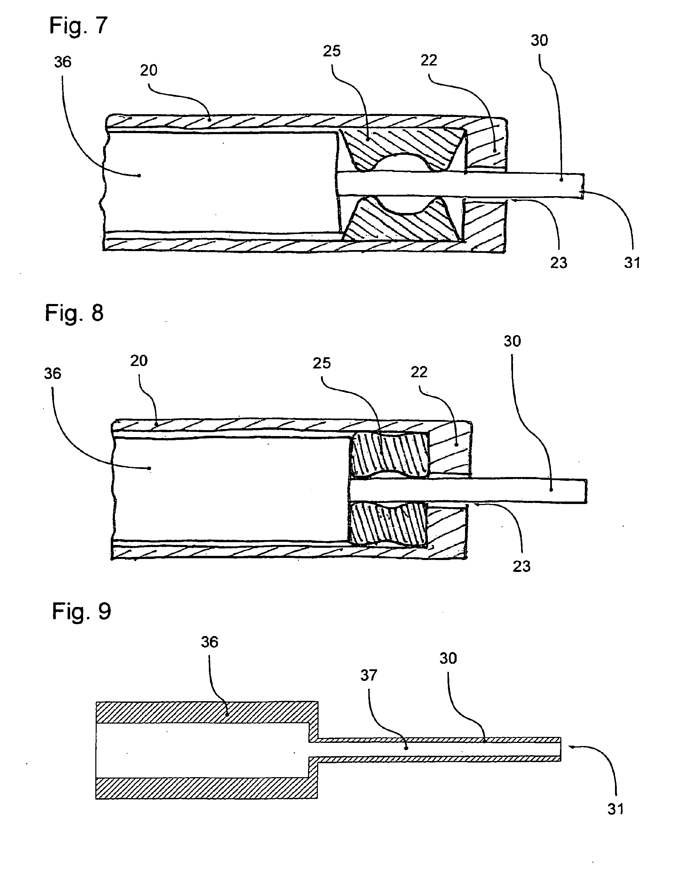

Fig. 7 und 8 zeigen den erfindungsgemäßen Schutzschlauches 20 mit entsprechender Führungseinrichtung 36 und Nadelelektrode 30. Ein Zwischenraum zwischen dem Abschlusselement 22 und der Führungseinrichtung 36 ist hier durch einen Elastomerring 25 ausgefüllt. Dieser Elastomerring 25 umgibt die Nadelelektrode 30 und ist mit dem Schutzschlauch 20 fest verbunden. Der Elastomerring 25 ist derart ausgebildet, dass er eine definierte Haftkraft induziert, die die Nadelelektrode 30 in der vorab eingestellten Position festsetzt. Der Elastomerring 25 bildet also ein Fixierelement, das die Nadelelektrode 30 am Distalende 21 des Schutzschlauches 20 umgreift. Der Elastomerring 25 kann derart ausgebildet sein, dass die entsprechende Position der Nadelelektrode 30 auch bei einem starken Abknicken des Schlauches 20 oder des Arbeitskanals 12, und bei entsprechend starken Bewegungen, eingehalten wird. Das Fixierelement induziert also eine Haftkraft, die bei einer Bewegung der Spitze 31 der Nadelelektrode relativ zum Schutzschlauch 20 überwunden werden muss. Die Fig. 8 zeigt die Nadelelektrode 30 in einer Position mit maximaler Nadellänge, die Fig. 7 in einer Position mit kurzer Nadellänge. Der Elastomerring 25 kann auch die Funktion eines Stopperelements und/oder die eines Federelements 34 und/oder des Abschlusselements 22 übernehmen. Des Weiteren kann der Elastomerring 25 derart ausgebildet sein, dass das Einführen der Nadelelektrode 31 in die Öffnung 23 erleichtert wird. Eine Kombination des Elastomerrings 25 der Fig. 7 und 8 mit der Spiralfeder der Fig. 3 und 4 ist möglich.

Die erfindungsgemäße Nadelelektrode 30 umfasst vorzugsweise, wie in Fig. 9 dargestellt, eine Kapillarleitung 37, die das Injizieren von Flüssigkeit mittels der Nadelelektrode 30 in Gewebeabschnitte ermöglicht. Bei einer ESD kann die erfindungsgemäße Nadelelektrode 30 somit eine Doppelfunktion übernehmen. Zum einen wirkt sie als Injektionsnadel zum Einbringen der Flüssigkeit 4 in das Gewebe, zum anderen als Elektrode zum Schneiden und Koagulieren des Gewebes.

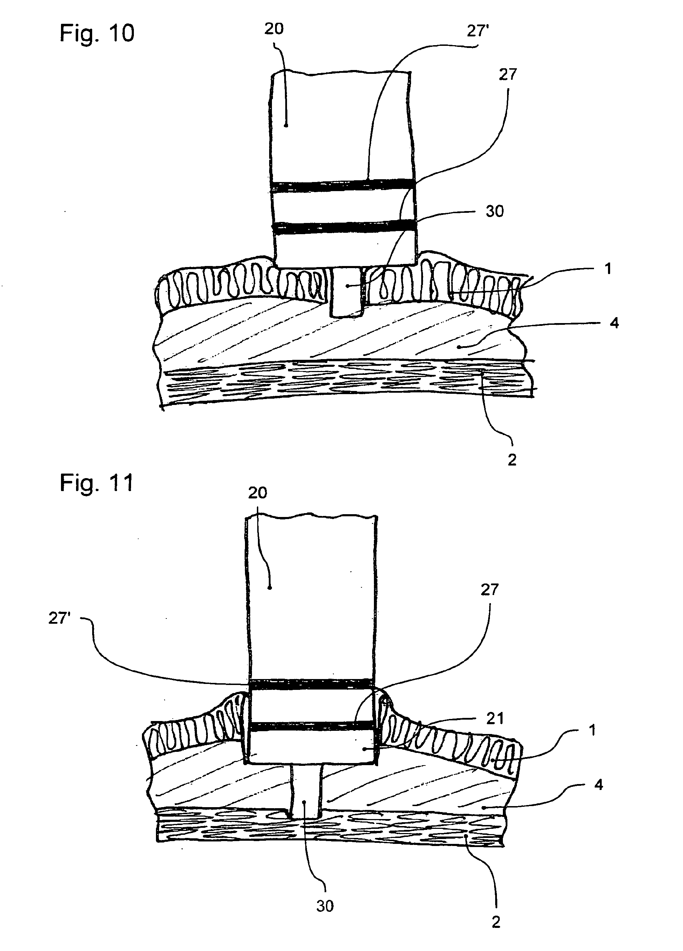

Fig. 10 und 11 zeigt eine Draufsicht auf den Schutzschlauch 20, wobei die Nadelelektrode 30 das Distalende 21 des Schutzschlauches 20 zumindest teilweise überragt. Das in Fig. 10 und 11 dargestellte Instrument wird zur Behandlung eines Hohlorgans eingesetzt.

Hierfür wurde für eine ESD Flüssigkeit 4 in einen Zwischenraum zwischen Mukosa 1 und Muskularis 2 eingebracht. Markierungen 27, 27' an der Außenwand des Schutzschlauches 20 nahe dem Distalende, helfen bei der Bestimmung der Position des Schutzschlauches 20 relativ zu den Gewebeschichten des Hohlorgans. Die Markierungen 27, 27' erstrecken sich rund um den Schutzschlauch 20 und haben einen definierten Abstand zum Distalende 21 des Schutzschlauchs. Die Markierungen 27, 27' sind derart ausgebildet, dass sie sich optisch leicht erfassen lassen. Die Markierungen 27, 27' helfen, ein übermäßig tiefes Eindringen des Schutzschlauches 20 in die Gewebeschichten des Hohlorgans zu vermeiden (vgl. Fig. 11). Alternativ kann anhand der Markierungen 27, 27' die Eindringtiefe des Distalendes 21 des Schutzschlauches 20 bestimmt werden und die Nadellänge entsprechend angepasst werden.

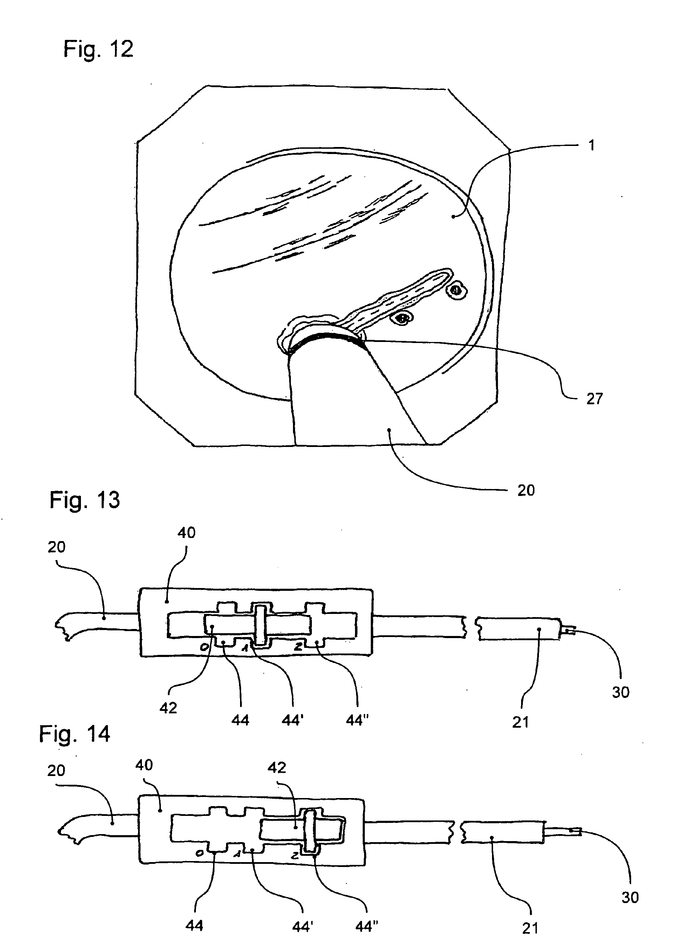

Die Fig. 12 stellt ein endoskopisches Bild des Schutzschlauches 20 dar. Die Tiefeneinschätzung, die bei endoskoptypischen Aufnahmen häufig schwierig ist, wird durch die Markierung 27 erleichtert. In Fig. 12 liegt das Distalende 21 des Schutzschlauches 20 auf der Mukosa 1 auf. Ein Schneiden des Gewebes wird durch die in der endoskopischen Darstellung nicht sichtbare Nadelelektrode 30 gewährleistet.

Die Fig. 13 und 14 zeigen eine erfindungsgemäße Bedieneinheit 40 zur Einstellung der Nadellänge der Nadelelektrode 30. Die Bedieneinheit 40 befindet sich am proximalen Ende des Schutzschlauches 20 und ist für den Arzt leicht zugänglich.

Die Bedieneinheit 40 umfasst einen Schieber 42 und mehrere Rasten 44, 44', 44". Der Schieber 42 steht in Wirkverbindung mit der Führungseinrichtung 36 (vgl. Fig. 3-6) und bestimmt somit indirekt die Position der Nadelelektrode 30. Ein Verschieben des Schiebers 42 in distaler Richtung führt zu einer Bewegung der Nadelelektrode 30 in distaler Richtung (vgl. Fig. 14), eine Bewegung des Schiebers 42 in proximaler Richtung führt zu einer Bewegung der Nadelelektrode 30 in proximaler Richtung (vgl. Fig. 13). Der Schieber 42 wirkt mit den Rasten 44, 44', 44" derart zusammen, dass sich die Nadelelektrode 30 in vorgegebenen Positionen festsetzen lässt. Hierfür rastet eine Ausbuchtung des Schiebers 42 entweder in einer ersten Raste 44, einer zweiten Raste 44' oder einer dritten Raste 44" ein. Vorzugsweise ist die Bedieneinheit 40 derart ausgestaltet, dass die Position des Schiebers 42 in der ersten Raste 44 (vgl. Beschriftung 0), der zweiten Raste 44' (vgl. Beschriftung 1) oder der dritten Raste 44" (vgl. Beschriftung 2) einer in den Schutzschlauch 20 zurück gezogenen Position der Nadelelektrode 30 bzw. einer teilweise ausgefahrenen Position der Nadelelektrode 30 bzw. einer maximal ausgefahrenen Position der Nadelelektrode 30 zugeordnet ist. Der Arzt muss sich also nicht auf die visuelle Kontrolle der Nadelposition verlassen, sondern kann seine Erkenntnisse mit den von der Bedieneinheit 40 vorgegebenen Einstellungen abgleichen. Es ist möglich und gewollt, die Bedieneinheit 40 gemäß den Fig. 13 und 14 in Kombination mit den vorab beschriebenen erfindungsgemäßen Schutzschlauch 20 einzusetzen.

### Bezugszeichenliste

- 1: Mukosa
- 2: Muskularis
- 4: Flüssigkeit
- 6: Geweberückstände
- 10: Endoskop
- 12: Arbeitskanal
- 20: Schutzschlauch
- 21: Distalende des Schutzschlauches
- 22: Abschlusselement
- 23: Öffnung
- 25: Elastomerring
- 27, 27': Markierung
- 30: Nadelelektrode
- 31: Spitze der Nadelelektrode
- 34: Federelement
- 36: Führungseinrichtung
- 37: Kapillarleitung
- 40: Bedieneinheit
- 42: Schieber
- 44,44',44": Raste
- d1: Innendurchmesser der Öffnung
- d2: Innendurchmesser des Schutzschlauches
- d3: Innendurchmesser der Führungseinrichtung

## Patentansprüche

1. Elektrochirurgisches Instrument, umfassend einen in einen Arbeitskanal (12) eines Endoskops (10) einführbaren Schutzschlauch (20) und eine Elektrode (30), die innerhalb des Schutzschlauches (20) mittels eines Bedienelements in eine distale Richtung und eine proximale Richtung bewegbar ist, wobei die Elektrode (30) durch eine Bewegung in die distale Richtung in eine ausgefahrene Position bringbar ist, in der die Elektrode (30) zumindest teilweise über ein distales Ende (21) des Schutzschlauches (20) herausragt,
wobei das distale Ende (21) des Schutzschlauches (20) ein Abschlusselement (22) mit einer Öffnung (23) für den Durchlass der Elektrode (30) umfasst, wobei die Öffnung (23) des Abschlusselements (22) zur Führung und/oder Reinigung der Elektrode (30) einen maximalen Innendurchmesser d1 aufweist, der geringer ist als der Innendurchmesser d2 des Schutzschlauches (20), dadurch charakterisiert, dass ein Fixierelement am distalen Ende (21) des Schutzschlauches (20) derart ausgebildet ist, dass das Fixierelement eine definierte Haftkraft induziert, die einer Bewegung der Elektrode (30) in der distalen und proximalen Richtung entgegen wirkt, wobei das Fixierelement mindestens ein elastisches Element umfasst, das innerhalb des Schutzschlauches (20) angeordnet ist.

2. Elektrochirurgisches Instrument nach Anspruch 1,
**dadurch gekennzeichnet, dass**
die Elektrode (30) ein Stopperelement umfasst, das mit dem Abschlusselement (22) derart zusammenwirkt, dass es die Bewegung der Elektrode (30) in die distale Richtung beschränkt.

3. Elektrochirurgisches Instrument nach Anspruch 2,
**dadurch gekennzeichnet, dass**
das Stopperelement im Wesentlichen zylindrisch zur kräftefreien Führung der Elektrode (30) innerhalb des Schutzschlauchs (20) ausgebildet ist.

4. Elektrochirurgisches Instrument nach Anspruch 2 oder 3,
**dadurch gekennzeichnet, dass**
ein Federelement (34) zwischen Stopperelement und Abschlusselement (22) angeordnet ist, das in der ausgefahrenen Position der Elektrode (30) eine Kraft in die proximale Richtung induziert.

5. Elektrochirurgisches Instrument nach einem der vorhergehenden Ansprüche,
**dadurch gekennzeichnet, dass**
die Öffnung (23) des Abschlusselements (22) dem Querschnitt der Elektrode (30) formentsprechend ausgebildet ist.

6. Elektrochirurgisches Instrument nach einem der vorhergehenden Ansprüche,
**dadurch gekennzeichnet, dass**
die Öffnung (23) des Abschlusselements (22) zur kraftfreien Durchführung der Elektrode (30) ausgebildet ist.

7. Elektrochirurgisches Instrument nach einem der vorhergehenden Ansprüche,
**dadurch gekennzeichnet, dass**
die Elektrode (30) eine Nadel umfasst.

8. Elektrochirurgisches Instrument nach einem der vorhergehenden Ansprüche,
**dadurch gekennzeichnet, dass**
die Elektrode (30) eine Kapillarleitung zum Injizieren von Flüssigkeit umfasst.

9. Elektrochirurgisches Instrument nach einem der vorhergehenden Ansprüche,
**dadurch gekennzeichnet, dass**
der Schutzschlauch (20) mindestens eine insbesondere ringförmig ausgebildete Markierung (27, 27') an der Außenseite des Schutzschlauches (20) nahe dessen distalem Ende (21) umfasst.

10. Elektrochirurgisches Instrument nach einem der vorhergehenden Ansprüche,
**gekennzeichnet durch**
eine Bedieneinheit zur Einstellung der Position der Elektrode (30) relativ zum distalen Ende (21) des Schutzschlauches (20).

11. Elektrochirurgisches Instrument nach Anspruch10,
**dadurch gekennzeichnet, dass**
die Bedieneinheit mindestens eine Sperreinrichtung umfasst, mit der das Bedienelement in mindestens einer Einstellposition festgesetzt werden kann.

12. Elektrochirurgisches Instrument nach Anspruch11,
**gekennzeichnet durch**
mindestens zwei Einstellpositionen, wobei in einer ersten Einstellposition die Elektrode (30) zumindest teilweise über ein distales Ende (21) des Schutzschlauches (20) herausragt und in einer zweiten Einstellposition die Elektrode (30) innerhalb des Schutzschlauches liegt.

13. Endoskop mit mindestens einem Arbeitskanal und einem elektrochirurgischem Instrument nach einem der vorhergehenden Ansprüche.

## Claims

1. Electrosurgical instrument, comprising a protection tube (20), which can be inserted into a work channel (12) of an endoscope (10), and an electrode (30), which can be moved in a distal direction and a proximal direction within the protection tube (20) by means of an operating element, wherein the electrode (30) can, by a movement in the distal direction, be brought into an extended position, in which the electrode (30) at least partly protrudes over a distal end (21) of the protection tube (20),
wherein the distal end (21) of the protection tube (20) comprises a termination element (22) with an opening (23) for passing the electrode (30) through, wherein the opening (23) of the termination element (22) for guiding and/or cleaning the electrode (30) has a maximum internal diameter d1, which is smaller than the internal diameter d2 of the protection tube (20), **characterized in that** a fixing element is formed at the distal end (21) of the protection tube (20) such that the fixing element induces a defined adhesive strength which counteracts a movement of the electrode (30) in the distal and proximal direction, wherein the fixing element comprises at least one elastic element that is arranged within the protection tube (20).

2. Electrosurgical instrument according to Claim 1,
**characterized in that**
the electrode (30) comprises a stopper element which interacts with the termination element (22) such that it restricts the movement of the electrode (30) in the distal direction.

3. Electrosurgical instrument according to Claim 2,
**characterized in that**
the stopper element is embodied substantially cylindrically for force-free guidance of the electrode (30) within the protection tube (20).

4. Electrosurgical instrument according to Claim 2 or 3,
**characterized in that**
arranged between stopper element and termination element (22) there is a spring element (34), which induces a force in the proximal direction in the extended position of the electrode (30).

5. Electrosurgical instrument according to one of the preceding claims,
**characterized in that**
the opening (23) of the termination element (22) is embodied with a shape corresponding to the cross section of the electrode (30).

6. Electrosurgical instrument according to one of the preceding claims,
**characterized in that**
the opening (23) of the termination element (22) is embodied for the force-free passage of the electrode (30).

7. Electrosurgical instrument according to one of the preceding claims,
**characterized in that**
the electrode (30) comprises a needle.

8. Electrosurgical instrument according to one of the preceding claims,
**characterized in that**
the electrode (30) comprises a capillary line for injecting liquid.

9. Electrosurgical instrument according to one of the preceding claims,
**characterized in that**
the protection tube (20) comprises at least one marking (27, 27'), more particularly with an annular design, on the outer side of the protection tube (20) in the vicinity of the distal end (21) thereof.

10. Electrosurgical instrument according to one of the preceding claims,
**characterized by**
an operating unit for adjusting the position of the electrode (30) relative to the distal end (21) of the protection tube (20).

11. Electrosurgical instrument according to Claim 10,
**characterized in that**
the operating unit comprises at least one locking apparatus, by means of which the operating element can be fixed in at least one adjustment position.

12. Electrosurgical instrument according to Claim 11,
**characterized by**
at least two adjustment positions, wherein, in a first adjustment position, the electrode (30) protrudes at least in part over a distal end (21) of the protection tube (20) and, in a second adjustment position, the electrode (30) is situated within the protection tube.

13. Endoscope with at least one work channel and one electrosurgical instrument according to one of the preceding claims.

## Revendications

1. Instrument électrochirurgical, comprenant un tuyau flexible de protection (20) pouvant être introduit dans un canal de travail (12) d'un endoscope (10) et une électrode (30) pouvant être déplacée à l'intérieur du tuyau flexible de protection (20) à l'aide d'un élément de commande dans une direction distale et une direction proximale, l'électrode (30) pouvant être amenée par le biais d'un mouvement réalisé dans la direction distale dans une position déployée dans laquelle l'électrode (30) ressort au moins en partie au-delà d'une extrémité distale (21) du tuyau flexible de protection (20) ;
l'extrémité distale (21) du tuyau flexible de protection (20) comprenant un élément terminal (22) doté d'une ouverture (23) pour le passage de l'électrode (30), l'ouverture (23) de l'élément terminal (22) comportant un diamètre intérieur maximal d1 pour le guidage et/ou le nettoyage de l'électrode (30), ledit diamètre étant plus réduit que le diamètre intérieur d2 du tuyau flexible de protection (20) ; **caractérisé en ce qu'**un élément de fixation situé au niveau de l'extrémité distale (21) du tuyau flexible de protection (20) est réalisé de telle sorte que l'élément de fixation induit une force d'adhérence définie agissant à l'encontre d'un déplacement de l'électrode (30) dans la direction distale et proximale, l'élément de fixation comprenant au moins un élément élastique disposé à l'intérieur du tuyau flexible de protection (20).

2. Instrument électrochirurgical selon la revendication 1, **caractérisé en ce que** l'électrode (30) comprend un élément de butée interagissant avec l'élément terminal (22) de telle sorte qu'il limite le déplacement de l'électrode (30) dans la direction distale.

3. Instrument électrochirurgical selon la revendication 2, **caractérisé en ce que** l'élément de butée est réalisé pour l'essentiel de façon cylindrique pour permettre de guider sans forces l'électrode (30) à l'intérieur du tuyau flexible de protection (20).

4. Instrument électrochirurgical selon la revendication 2 ou 3, **caractérisé en ce qu'**un élément de ressort (34) est disposé entre l'élément de butée et l'élément terminal (22), ledit élément de ressort induisant une force dans la direction proximale dans la position déployée de l'électrode (30).

5. Instrument électrochirurgical selon l'une quelconque des revendications précédentes, **caractérisé en ce que** l'ouverture (23) de l'élément terminal (22) est réalisée par complémentarité de formes par rapport à la section transversale de l'électrode (30).

6. Instrument électrochirurgical selon l'une quelconque des revendications précédentes, **caractérisé en ce que** l'ouverture (23) de l'élément terminal (22) est réalisée de façon à laisser passer sans force l'électrode (30).

7. Instrument électrochirurgical selon l'une quelconque des revendications précédentes, **caractérisé en ce que** l'électrode (30) comprend une aiguille.

8. Instrument électrochirurgical selon l'une quelconque des revendications précédentes, **caractérisé en ce que** l'électrode (30) comprend une conduite capillaire pour injecter un fluide.

9. Instrument électrochirurgical selon l'une quelconque des revendications précédentes, **caractérisé en ce que** le tuyau flexible de protection (20) comprend au moins un marquage (27, 27') prenant notamment une forme annulaire réalisé au niveau du côté extérieur du tuyau flexible de protection (20) à proximité de son extrémité distale (21).

10. Instrument électrochirurgical selon l'une quelconque des revendications précédentes, **caractérisé par** une unité de commande servant au réglage de la position de l'électrode (30) par rapport à l'extrémité distale (21) du tuyau flexible de protection (20).

11. Instrument électrochirurgical selon la revendication 10, **caractérisé en ce que** l'unité de commande comprend au moins un dispositif de blocage permettant de fixer l'élément de commande dans au moins une position de réglage.

12. Instrument électrochirurgical selon la revendication 11, **caractérisé par** au moins deux positions de réglage, l'électrode (30) ressortant au moins en partie au-delà d'une extrémité distale (21) du tuyau flexible de protection (20) dans une première position de réglage et l'électrode (30) se trouvant à l'intérieur du tuyau flexible de protection dans une deuxième position de réglage.

13. Endoscope équipé d'au moins un canal de travail et d'un instrument électrochirurgical selon l'une quelconque des revendications précédentes.
